(19) **Europäisches Patentamt**

**European Patent Office**

**Office européen des brevets**

(11) **EP 0 773 035 B2**

(12) **NEUE EUROPÄISCHE PATENTSCHRIFT**

(45) Veröffentlichungstag und Bekanntmachung des Hinweises auf die Entscheidung über den Einspruch:
**27.09.2006 Patentblatt 2006/39**

(45) Hinweis auf die Patenterteilung:
**19.02.2003 Patentblatt 2003/08**

(21) Anmeldenummer: **96117473.7**

(22) Anmeldetag: **31.10.1996**

(51) Int Cl.:
***A61M 1/36*** (2006.01)

(54) **Vorrichtung zur Ermittlung hämodynamischer Parameter während einer extrakorporalen Blutbehandlung**

Apparatus for determining hemodynamic variables during extracorporal blood treatment

Dispositif pour déterminer des variables hémodynamiques pendant un traitement extracorporel du sang

(84) Benannte Vertragsstaaten:
**DE ES FR GB IT**

(30) Priorität: **09.11.1995 DE 19541783**

(43) Veröffentlichungstag der Anmeldung:
**14.05.1997 Patentblatt 1997/20**

(73) Patentinhaber: **Fresenius Medical Care Deutschland GmbH**
**61352 Bad Homburg v.d.H. (DE)**

(72) Erfinder: **Krämer, Matthias, Dr.**
**61381 Friedrichsdorf (DE)**

(74) Vertreter: **Luderschmidt, Schüler & Partner Patentanwälte,**
**Postfach 3929**
**65029 Wiesbaden (DE)**

(56) Entgegenhaltungen:
**EP-A- 0 590 810       DE-A- 3 817 603**
**US-A- 5 312 550       US-A- 5 588 959**

- **Kaufmann, Kramer et al., Hemodialysis Access Recirculation Measurement by Blood Temperature monitoring (BTM)-A New Technique, J Am. soc. Nephtol 2.332 1991;**
- **Kraemer, Polaschegg, Control of blood Temperature and Thermal Energy Balance during Hemodialysis. Proceedings of the Annual International Conference of the IEEE Engineering in Medicine and Biology Society 14, Seite 2299, October 1992**
- **Krivitski, Theory and validation of access flow measurement by dilution technique during hemodialysis, Kidney International, 48 seite 245-250 1995;**
- **Depner, Krivitski, MacGibbon, Hemiodyalysis Access Recirculation Measured by Ultrasound Dilution, ASAIO Journal 41(3) M749-M753 July, 1995;**
- **Depner, Techniques for Prospective Detection of Venous Stenosis, Advances in Renal Replacement Therapy 2(2) Seiten 119-130, 1994;**
- **Aldridge C., Greenwood R.N., Cattel W.R.; The assessment of arteriovenous fistulae created for haemodialysis from pressure and thermal dilution measurements; Journal of Medical engineering & Technology 8(3) seiten 118-124, 1984;**
- **Guyton A.C. and Hall J. E., Textbook of Medical Physiology; Pub: Saunders, Seiten 250-251;**
- **Aldrige C., greenwood R. N., Frampton C. F., Wilkinson J. S. Cattel W. R.; Instument Design for the Bedside Assessment of Arteriovenous Fistulae in Haemodialysis Patients; Proc EDNTA-ERCA (1985) Vol 14, Seiten 255-260**

EP 0 773 035 B2

**Beschreibung**

Die Erfindung betrifft eine Vorrichtung zur Ermittlung hämodynamischer Parameter während einer extrakorporalen Blutbehandlung.

Bei Verfahren der chronischen Blutreinigungstherapie wie Hämodialyse, Hämofiltration und Hämodiafiltration wird Blut über einen extrakorporalen Kreislauf geleitet. Als Zugang zum Blutgefäßsystem wird häufig operativ eine arteriovenöse Fistel angelegt. Ebenso ist der Einsatz eines Implantats möglich. Wenn nachfolgend von dem Begriff "Fistel" die Rede ist, wird darunter jede Art der Verbindung zwischen einer Vene und einer Arterie des Patienten verstanden.

Der Gefäßzugang sollte einen Blutfluß liefern, der mindestens so groß ist, wie der extrakorporale Blutfluß, der durch die im extrakorporalen Kreislauf vorhandene Pumpe vorgegeben ist. Ist dies nicht der Fall, z.B. infolge von Verengungen (Stenosen) der Gefäße, kann sich die arterielle Nadel an der Gefäßwand ansaugen, was eine Unterbrechung des extrakorporalen Kreislaufs bewirkt. In den meisten Fällen wird aber ein Teil des extrakorporalen Blutflusses, nämlich die Differenz aus dem extrakorporalen Blutfluß und dem Blutfluß des in die Fistel strömenden Blutes, im extrakorporalen Kreislauf rezirkulieren. Diese Erscheinung wird als Rezirkulation bezeichnet.

Die Fistelrezirkulation hat zur Folge, daß die dem Körper pro Zeiteinheit entzogene Menge an dialysepflichtigen Substanzen verringert wird. Der rezirkulierende Anteil passiert nicht das Kapillarsystem des Körpers und wird folglich nicht erneut mit toxischen Substanzen beladen. Dieser Anteil trägt daher nur vermindert zur Blutreinigung bei. Wird eine durch die Rezirkulation bedingte erhebliche Verminderung der Dialyseeffektivität nicht erkannt und kompensiert, wird langfristig ein Anstieg der Morbidität solcher Patienten erfolgen. Die Messung der Qualität des Gefäßzugangs ist damit ein wichtiges Mittel zur Qualitätssicherung bei der Dialysebehandlung.

Es sind verschiedene Verfahren zur Messung der Fistelrezirkulation bekannt. Allen gemeinsam ist die Messung einer physikalisch-chemischen Eigenschaft des Blutes, die im venösen Blut veränderbar sein muß. Die physikalischchemische Eigenschaft kann durch direkte Aktion des Anwenders oder mittelbar über die Dialysataufbereitungseinheit geändert werden. Das Auftreten von Fistelrezirkulation kann anschließend festgestellt bzw. quantifiziert werden, indem eine Änderung dieser Eigenschaft auch im arteriellen Blut nachgewiesen wird.

US-A-5,312,550 und EP 0 590 810 A1 beschreiben ein derartiges Verfahren, bei dem eine Indikatorlösung in die venöse Leitung injiziert und deren Konzentration im arteriellen Blut überwacht wird. Die Injektion einer Indikatorlösung kann auch dadurch umgangen werden, daß ein kurzzeitiger Temperaturabfall im Dialysierflüssigkeitskreislauf erzeugt wird, der sich auf den venösen Zweig des extrakorporalen Kreislaufs überträgt und zu einem nachweisbaren Temperatursprung im arteriellen Zweig des extrakorporalen Kreislaufs dann führt, wenn Fistelrezirkulation vorliegt (M. Krämer und H.D. Polaschegg, EDTNA-ERCA J. 19, 6 (1993)).

Die Interpretation der gemessenen Rezirkulation ist jedoch nicht immer ganz einfach, da dieser technische Parameter mit dem eigentlich interessierenden physiologischen Parameter, nämlich dem Blutfluß zur Fistel $Q_F$, nicht in einfacher Weise korreliert ist. So variiert z.B. die Rezirkulation trotz konstantem Fistelfluß $Q_F$ mit dem Blutfluß $Q_B$. Außerdem erfassen viele Meßverfahren nicht nur die Fistelrezirkulation, sondern die Summe aus Fistel und kardiopulmonärer Rezirkulation (M. Krämer und H.D. Polaschegg, EDTNA-ERCA J. 19, 6 (1993)). Die kardiopulmonäre Rezirkulation, die als fraktioneller Anteil des Fistelflusses am Herzminutenvolumen definiert ist, betrifft bereits dialysiertes Blut, das über den Herz-/Lungenkreislauf direkt in den arteriellen Zweig des extrakorporalen Kreislaufs gelangt, ohne das Kapillarsystem zu durchlaufen. Auf die kardiopulmonäre Rezirkulation ist die Änderung der Bluttemperatur in der arteriellen Blutleitung vor Einsetzen der Fistelrezirkutation zurückzuführen. Die Überlagerung der Fistelrezirkulation und der kardiopulmonären Rezirkulation erschwert zusätzlich die Interpretation der Meßergebnisse.

Wünschenswert ist daher eine direkte Messung des Fistelflusses QF. Erste Ansätze dazu sind in einer Veröffentlichung von Aldridge et al., Journal of Medical Engineering and Technology 8, 118 (1984) beschrieben. Es werden einzelne Rezirkulationsmessungen mit der Thermodilutionsmethode nach Injektion eines Bolus kalter Kochsalzlösung durchgeführt. Die Messungen werden bei schrittweise erhöhtem Blutfluß solange wiederholt, bis eine merkliche Rezirkulation festgestellt wird. Bei diesem Blutfluß ist dann der Fistelfluß $Q_F$ überschritten. Problem dieses Verfahrens ist, daß der Fistelfluß nur auf das Meßintervall eingegrenzt und damit nicht genau bestimmt wird und daß die Messung wegen der fortwährenden Bolusinjektionen aufwendig ist. Vor allem aber wird in dieser Arbeit nicht berücksichtigt, daß in vivo bei jedem Blutfluß eine Rezirkulation vorhanden ist, nämlich die kardiopulmonäre Rezirkulation, die sich der Fistelrezirkulation überlagert. Aldridge et al. geben noch eine zweite Methode an, die zur Detektion des Fistelflusses geeigneter erscheint. Wenn bei Variation des extrakorporalen Blutflusses $Q_B$ dieser sehr nahe am Fistelfluß $Q_F$ liegt, wird eine Oszillation der arteriellen Temperatur TA beobachtet. Diese resultiert aus den periodisch schwankenden Druckverhältnissen in der Fistel durch die Aktion der Blutpumpe, die üblicherweise als Rollenpumpe ausgebildet ist. Nachteil dieses Verfahrens ist, daß der "richtige" Blutfluß $Q_B = Q_F$ nur langwierig durch Variation von $Q_B$ und Prüfen von TA auf das Vorliegen von Oszillationen gefunden werden kann. Vor allem aber muß die Temperatursensorik im arteriellen System eine sehr niedrige Ansprechzeit aufweisen, da die Periode der Oszillationen im Bereich einiger Zehntel Sekunden bis etwa einer Sekunde liegt. Diese Anforderung kann i.A. nur durch direktes Plazieren der Sensoren im Blutstrom erreicht werden, was für die Routinebehandlung nicht tragbar ist.

Der Erfindung liegt die Aufgabe zugrunde eine Vorrichtung zu schaffen, mit der sich hämodynamische Parameter während einer extrakorporalen Blutbehandlung mit großer Zuverlässigkeit ermitteln lassen.

Die Lösung dieser Aufgabe erfolgt erfindungsgemäß mit den Merkmalen des Patentanspruchs 1.

Bei der erfindungsgemäßen Vorrichtung zur Ermittlung hämodynamischer Parameter wie Fistelfluß, Herzminutenvolumen und für den Fall der Temperaturmessung die Körpertemperatur wird eine physikalische oder chemische Kenngröße des Blutes im arteriellen Zweig des extrakorporalen Kreislaufs gemessen. Es kann jede beliebige physikalische oder chemische Größe gemessen werden, die folgende Bedingungen erfüllt. Sie muß im venösen, d.h. in dem aus dem extrakorporalen Kreislauf in den venösen Teil der Fistel des Patienten zurückfließenden Blut einen anderen Wert als in dem zur Fistel fließenden Blut haben. Der Wert der physikalischen oder chemischen Kenngröße in einer Mischung aus zwei Teilvolumina Blut ($V_1$ und $V_2$) muß sich in analoger Weise wie für die Temperatur T mit hinreichender Genauigkeit nach der folgenden Mischungsgleichung ergeben:

$$V_1 \cdot T_1 + V_2 \cdot T_2 = (V_1 + V_2) \cdot T_M,$$

wobei $V_{1/2}$ die Teilvolumina und $T_{1/2}$ die Werte der zu messenden physikalischen oder chemischen Kenngröße in den beiden Teilvolumina sind und $T_M$ der Wert nach der Mischung ist.

Als physikalische oder chemische Kenngröße können neben der Temperatur auch die Konzentration eines Blutbestandteils, der Hämatokrit, die Dichte, die Ausbreitungsgeschwindigkeit des Schalls, die optische Dichte, die Leitfähigkeit oder die Viskosität gemessen werden. Diese Größen lassen sich mit bekannten Meßfühlern ermitteln.

Vorteilhaft ist, wenn als physikalische oder chemische Kenngröße die Bluttemperatur gemessen wird. Wenn die Bluttemperatur gemessen wird, lassen sich mit dem erfindungsgemäßen Verfahren neben dem Fistelfluß und dem Herzminutenvolumen auch die Körpertemperatur bestimmen, worunter die mittlere Temperatur des Bluts nach Durchfließen aller Kapillarsysteme verstanden wird. Eine Injektion einer Indikatorlösung ist dann nicht erforderlich.

Die erfindungsgemäße Vorrichtung beruht darauf" daß die physikalische oder chemische Kenngröße, vorzugsweise die Bruttemperatur, im venösen Zweig des extrakorporalen Kreislaufs während der Aufnahme der Meßwerte konstant gehalten ist. Ferner setzt das erfindungsgemäße Verfahren voraus, daß die physikalische oder chemische Kenngröße im venösen Zweig ihrer Größe nach bekannt ist. Für den Fall, daß die physikalische oder chemische Kenngröße weder konstant noch ihrer Größe nach bekannt sein sollte, muß diese gemessen und während der Aufnahme der Meßwerte konstant gehalten werden.

Die erfindungsgemäße Vorrichtung beruht darauf, daß sich die in diskreten Meßwerten vorliegende Meßkurve durch zwei Teilfunktionen darstellen läßt, wobei die erste Teilfunktion die physikalische oder chemische Kenngröße in Abhängigkeit vom extrakorporalen Blutfluß für Blutflußwerte kleiner als der Fistelfluß oder gleich dem Fistelfluß angibt und die zweite Teilfunktion die physikalische oder chemische Kenngröße in Abhängigkeit vom Blutfluß für Blutflußwerte größer oder gleich dem Fistelfluß angibt. Der Schnittpunkt beider Teilfunktionen gibt den Punkt an, an dem der extrakorporale Blutfluß gleich dem Fistelfluß ist. Aus dem "Knickpunkt" des Funktionsverlaufs, d.h. der Diskontinuität der Kurvensteigung läßt sich also der Punkt bestimmen, an dem die Fistelrezirkulation einsetzt, d.h. der Blutfluß gleich dem Fistelfluß ist.

Zur Bestimmung des Fistelflusses wird aus der abgespeicherten Folge von Wertepaaren der physikalischen oder chemischen Kenngröße des Blutes im arteriellen Zweig des extrakorporalen Kreislaufs derjenige Wert des Blutflusses bestimmt, nach dessen Überschreiten der Betrag der Änderung der physikalischen oder chemischen Kenngröße in einem bestimmten Blutflußintervall, d.h. der Betrag der Steigung der die Meßwertpaare darstellenden Funktion größer als ein vorgegebener Grenzwert ist. Die Steigungen in den einzelnen Meßintervallen können z.B. durch Berechnung der Differenzenquotienten der Meßwertpaare bestimmt werden. Der ermittelte Blutflußwert stellt einen Schätzwert für den Fistelfluß dar.

Für den Fall, daß der Fistelfluß mit sehr großer Genauigkeit bestimmt werden soll, wird der in der Folge der Wertepaare vorliegende Funktionsverlauf für Blutflußwerte kleiner als der Schätzwert für den Fistelfluß durch eine erste Teilfunktion dargestellt, während der in der Folge der Wertepaare vorliegende Funktionsverlauf für Blutflußwerte größer als der Schätzwert durch eine zweite Teilfunktion dargestellt wird.

Der exakte Wert des Fistelflusses wird dann aus dem Schnittpunkt der beiden Teilfunktionen ermittelt. Die Teilfunktion für Blutflußwerte kleiner oder gleich dem Fistelfluß kann näherungsweise durch eine lineare Gleichung dargestellt werden.

Der mittlere Wert der physikalischen oder chemischen Kenngröße im venösen Blut des Patienten ergibt sich durch Extrapolation des Funktionsverlaufes für Blutflußwerte kleiner oder gleich dem Fistelfluß auf einen Blutflußwert von Null. Im Falle einer Temperaturmessung läßt sich die Körpertemperatur bestimmen. Nachdem der Fistelfluß und der mittlere Wert der physikalisch-chemischen Eigenschaft ermittelt sind, kann das Herzminutenvolumen berechnet werden.

Die erfindungsgemäße Vorrichtung zur Ermittlung der hämodynamischen Parameter weist eine arterielle Meßeinrichtung zum Messen der physikalisch-chemischen Eigenschaft im arteriellen Zweig des extrakorporalen Kreislaufs, und eine Steuereinheit zum Verändern der Förderrate der Blutpumpe auf. Ferner ist eine Speichereinheit zum Abspeichern der Werte der physikalisch-chemischen Eigenschaft und des extrakorporalen Blutflusses und eine Recheneinheit zur Ermittlung der hämodynamischen Parameter aus den abgespeicherten Wertepaaren vorgesehen. Die erfindungsgemäße Vorrichtung kann in die bekannten Blutreinigungsvorrichtungen integriert werden, wobei auf bereits vorhandene Komponenten, über die die bekannten Blutbehandlungsvorrichtungen bereits verfügen, zurückgegriffen werden kann.

Für den Fall, daß die physikalische oder chemische Kenngröße im venösen Zweig des extrakorporalen Kreislaufs nicht konstant sein sollte, weist die erfindungsgemäße Vorrichtung vorteilhafterweise eine Regeleinrichtung auf, die die physikalische oder chemische Kenngröße im venösen Zweig konstant hält. Diese kann z.B. im Falle einer Temperaturmessung als Temperaturregeleinrichtung ausgebildet sein.

Nachfolgend wird unter Bezugnahme auf die Zeichnungen ein Ausführungsbeispiel der erfindungsgemäßen Vorrichtung zur Ermittlung hämodynamischer Parameter näher erläutert.

Es zeigen:

Fig. 1      die erfindungsgemäße Vorrichtung zur Ermittlung hämodynamischer Parameter zusammen mit einer Dialysevorrichtung in schematischer Darstellung einschließlich des Intrakorporalen Kreislaufs,

Fig. 2      die Temperatur im arteriellen Zweig des extrakorporalen Kreislaufs als Funktion des extrakorporalen Blutflusses, und

Fig. 3      das Verhältnis der Steigungen der beiden Kurvenabschnitte der ersten und zweiten Teilfunktion zur Darstellung des Meßkurvenverlaufs für einen dem Fistelfluß entsprechenden extrakorporalen Blutfluß.

Die erfindungsgemäße Vorrichtung zur Ermittlung hämodynamischer Parameter kann eine separate Baugruppe bilden. Sie kann aber auch Bestandteil einer Dialysevorrichtung sein, zumal einige Komponenten der erfindungsgemäßen Vorrichtung in den bekannten Dialysevorrichtungen bereits vorhanden sind. Nachfolgend wird die erfindungsgemäße Vorrichtung zusammen mit den wesentlichen Komponenten der Dialysevorrichtung beschrieben. In dem Ausführungsbeispiel wird als physikalische oder chemische Kenngröße $X_A$ die Temperatur $T_A$ im arteriellen Zweig des extrakorporalen Kreislaufs gemessen, so daß sich neben dem Fistelfluß $Q_F$, die Körpertemperatur $T_B$, d.h. die mittlere Temperatur des Blutes nach Durchfließen aller Kapillarsysteme, und das Herzminutenvolumen CO ermitteln lassen.

Der intrakorporale Kreislauf 1 umfaßt das rechte Ventrikel 2 des Herzens, die Lunge 3, das linke Ventrikel 4 und sämtliche Kapillarsysteme des Körpers in inneren Organen, Muskulatur und Haut 5 etc.. Um einen Zugang zu dem Blutgefäßsystem zu schaffen, ist eine arteriovenöse Fistel 6 angelegt.

Die Dialysevorrichtung 7 besteht Im wesentlichen aus einem Dialysierfüssigkeitsteil 8 und einem extrakorporalen Blutkreislauf 9, zwischen denen sich ein Dialysator 10 mit einem Dialysierflüssigkeitskompartiment 11 und einem Blutkompartement 12 befindet. Das Dialysierflüssigkeitskompartment 11 ist stromauf des Dialysators 10 über eine Dialysierflüssigkeitsleitung 13 mit einer Dialysierflüssigkeitsquelle 14 verbunden. In die Dialysierflüssigkeitsleitung 13 ist eine Temperiereinrichtung 15 geschaltet, die über eine Steuerleitung 16 mit einer Regeleinrichtung 17 in Verbindung steht. Stromab des Dialysators 10 ist an das Dialysierflüssigkeitskompartment 11 eine weitere Leitung 18 angeschlossen, die eine Dialysierflüssigkeitspumpe 19 aufweist.

Der extrakorporale Kreislauf 9 umlaßt einen arteriellen Zweig 20, der mit dem arteriellen Teil 21 der Fistel 6 in Verbindung steht, das Blutkompartiment 12 des Dialysators 10 und einen venösen Zweig 22, der mit dem venösen Teil 23 der Fistel 6 in Verbindung steht. Im arteriellen Zweig 20 und im venösen Zeig 22 des extrakorporalen Kreislaufs ist jeweils eine Temperaturmeßeinrichtung 24, 25 zur Messung der arteriellen Fisteltemperatur, d.h. der Bluttemperatur nach Eintritt in den arteriellen Zweig 20 des extrakorporalen Kreislaufs 9 bzw. zur Messung der venösen Fisteltemperatur, d.h. der Bluttemperatur nach Eintritt in den venösen Zweig 22 des extrakorporalen Kreislaufs vorgesehen.

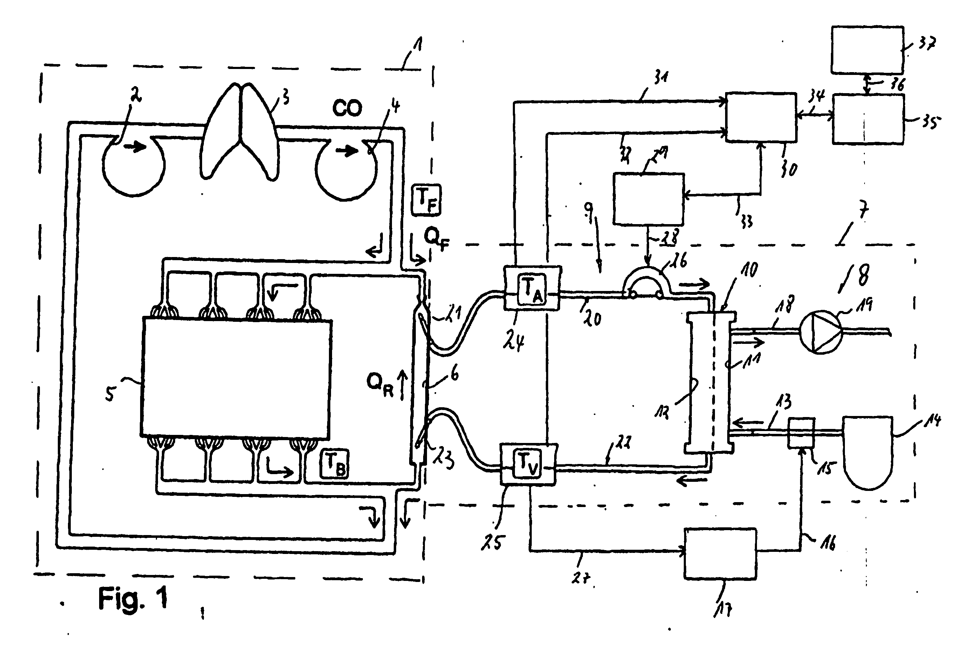

Ferner ist im arteriellen Zweig 20 des extrakorporalen Kreislaufs 9 eine Blutpumpe 26 angeordnet. Die venöse Temperaturmeßeinrichtung 25 ist über eine Leitung 27 an die Regeleinrichtung 17 angeschlossen. Die Regeleinrichtung 17 steuert die Temperiereinrichtung 15 im Dialysierflüssigkeitsteil 8 der Dialysevorrichtung 7 derart an, daß die Temperatur im venösen Zweig 22 des extrakorporalen Kreislaufs 9 konstant gehalten wird. Weitere bei einer Dialysevorrichtung gewöhnlich vorhandene Komponenten, wie Tropfkammem und Absperrklemmen sind in Fig. 1 nicht dargestellt.

Die Blutpumpe 26 im arteriellen Zweig 20 des extrakorporalen Kreislaufs ist über eine Steuerleitung 28 mit einer Steuereinheit 29 verbunden, mit der die Förderrate der Blutpumpe innerhalb bestimmter Bereiche verändert werden kann. Ferner ist eine Speichereinheit 30 vorgesehen, die über eine Datenleitung 31 die Meßwerte der arteriellen Temperaturmeßeinrichtung 24 empfängt und in zeitlicher Abfolge abspeichert. Ferner speichert die Speichereinheit 30 über eine Datenleitung 32 den konstanten Temperaturwert der venösen Temperaturmeßeinrichtung 25 ab. Die Speichereinheit 30 ist über eine Leitung 33 mit der Steuereinheit 29 verbunden. Über die Leitung 33 empfängt die Speichereinheit 30 den der eingestellten Förderrate der Blutpumpe 26 entsprechenden Wert des extrakorporalen Blutflusses und speichert diesen ab. Die Speichereinheit 30 steht über eine Datenleitung 34 mit einer Recheneinheit 35 in Verbindung, die ihrerseits über eine Datenleitung 36 mit einer Anzeigeeinheit 37 zur Anzeige der ermittelten hämodynamischen Parameter verbunden ist. Die Recheneinheit kann als bekannter Digitalrechner ausgebildet sein.

Nachfolgend wird das Prinzip der Messung im einzelnen erläutert.

Das vom linken Ventrikel 4 emittierte Blut fließt zum größten Teil in die Kapillarsysteme aller Organe, zu einem kleinen Teil in die Fistel. Für den Fall, daß der Blutfluß im extrakorporalen Kreislauf kleiner als der Blutfluß des in die Fistel bzw. aus der Fistel fließenden Blutes ist, fließt das Fistelblut zum einen Teil durch den extrakorporalen Kreislauf 9, zum anderen Teil durch die Fistel 6. Wenn der extrakorporale Blutfluß jedoch größer als der Fistelfluß ist, dann rezirkuliert Blut aus dem extrakorporalen Kreislauf 9, wobei die Fistel 6 vom venösen 23 zum arteriellen Anschluß 21 durchflossen wird. Das extrakorporale Blut, das durch die Fistel 6 fließende Blut und das aus den Kapillarsystemen stammende Blut vereinigt sich schließlich wieder im Rücklauf zum Herzen.

Die Temperaturen und Flüsse werden wie folgt bezeichnet:

$Q_F$    Fistelfluß, d.h. der Fluß in die Fistel 6 bzw. aus der Fistel heraus,

$Q_B$    Blutfluß im extrakorporalen Kreislauf 9,

$Q_R$    Rezikulationsfluß, d.h. der Fluß zwischen dem venösen Teil 23 und dem arteriellen Teil 21 der Fistel 6, falls $Q_F \leq Q_B$ ist,

CO    Herzminutenvolumen,

$T_B$    Körpertemperatur, d.h. die mittlere Temperatur des Blutes nach Durchfließen der Kapillarsysteme,

$T_F$    Fisteltemperatur, d.h. die Temperatur des in die Fistel 6 fließenden Blutes,

$T_A$    arterielle Fisteltemperatur, d.h. Bluttemperatur nach Eintritt in den arteriellen Zweig 20 des extrakorporalen Kreislaufs,

$T_V$    venöse Fisteltemperatur, d.h. Bluttemperatur nach Eintritt in den venösen Zweig 22 des extrakorporalen Kreislaufs.

Die erfindungsgemäße Vorrichtung beruht darauf, daß sich die gemessene Bluttemperatur $T_A$ im arteriellen Zweig des extrakorporalen Kreislaufs in Abhängigkeit von dem extrakorporalen Blutfluß $Q_B$ durch zwei Teilfunktionen darstellen läßt, wobei die eine Teilfunktion die arterielle Bluttemperatur $T_A$ für einen extrakorporalen Blutfluß kleiner oder gleich dem Fistelfluß angibt und die andere Teilfunktion die arterielle Bluttemperatur $T_A$ bei einem extrakorporalen Blutfluß größer oder gleich dem Fistelfluß angibt. Die beiden Teilfunktionen lassen sich wie folgt herleiten, wobei davon ausgegangen wird, daß die Ultrafiltration während der Messung abgeschaltet ist.

Es sei angenommen, daß der Blutfluß $Q_B$ im extrakorporalen Kreislauf 9 kleiner oder gleich dem Fistelfluß $Q_F$ ist. Das ins rechte Herz fließende Blut setzt sich aus den drei folgenden Komponenten zusammen, nämlich aus dem aus dem Kapillarsystem zum Herz zurückfließenden Blut, aus dem aus dem extrakorporalen Kreislauf 9 stammenden Blut und aus dem durch die Fistel 6, jedoch nicht durch den extrakorporalen Kreislauf fließenden Blut. Daraus ergibt sich für die Temperaturen folgende Mischungsgleichung:

$$(CO - Q_F) \cdot T_B + Q_B \cdot T_V + (Q_F - Q_B) \cdot T_F = CO \cdot T_F$$

Da das arterielle Blut nur aus einer Komponente, nämlich dem zur Fistel 6 fließenden Blut besteht, ist die arterielle Fisteltemperatur $T_A$ gleich der Fisteltemperatur $T_F$. Damit erhält man die folgende Teilfunktion:

$$T_A(Q_B) = \frac{(CO - Q_F) \cdot T_B + Q_B \cdot T_V}{CO - Q_F + Q_B} \tag{1}$$

Die obige Gleichung läßt sich auch wie folgt darstellen:

$$T_A(Q_B) = (\gamma + \delta Q_B)/(\varepsilon + Q_B)$$

mit

$$\gamma = (CO-Q_F)T_B, \ \delta = T_V, \ \varepsilon = CO-Q_F \qquad (1)$$

Es sei angenommen, daß der Blutfluß $Q_B$ im extrakorporalen Kreislauf 9 größer als der Fistelfluß $Q_F$ ist. In diesem Fall setzt sich das in den arteriellen Zweig 20 des extrakorporalen Kreislaufs 9 einfließende Blut zusammen aus dem in die Fistel 6 strömenden Blut und dem über die Fistel rezirkulierenden, d.h. aus dem venösen Zweig 22 des extrakorporalen Kreislaufs stammenden Blut. Daraus ergibt sich für die Temperaturen folgende Mischungsgleichung:

$$Q_F \cdot TF + Q_R \cdot T_V = Q_B \cdot T_A$$

Das Fistelblut setzt sich wie folgt zusammen aus dem Kapillarblut und dem aus dem extrakorporalen Kreislauf 9 stammenden Blut.

$$(CO - Q_F) \cdot T_B + Q_F \cdot T_V = CO \cdot T_F$$

Aus diesen beiden Gleichungen folgt mit $Q_R = Q_B - Q_F$ die zweite Teilfunktion:

$$T_A(Q_B) = \left(1 - \frac{Q_F}{CO}\right) \cdot \frac{Q_F}{Q_B} \cdot T_B + \frac{Q_F^2}{Q_B \cdot CO} \cdot T_V + \left(1 - \frac{Q_F}{Q_B}\right) \cdot T_V \qquad (2)$$

Die zweite Teilfunktion läßt sich auch wie folgt darstellen:

$$T_A(Q_B) = \alpha/Q_B + \beta$$

mit

$$\alpha = Q_F T_B(1 - Q_F/CO) + Q_F^2 T_V/CO - Q_F T_V, \ \beta = T_V \qquad (2)$$

Die Funktion $T_A(Q_B)$ besteht also aus den beiden Teilfunktionen (1) und (2), jeweils für die Bereiche $Q_F \geq Q_B$ und $Q_F < Q_B$.

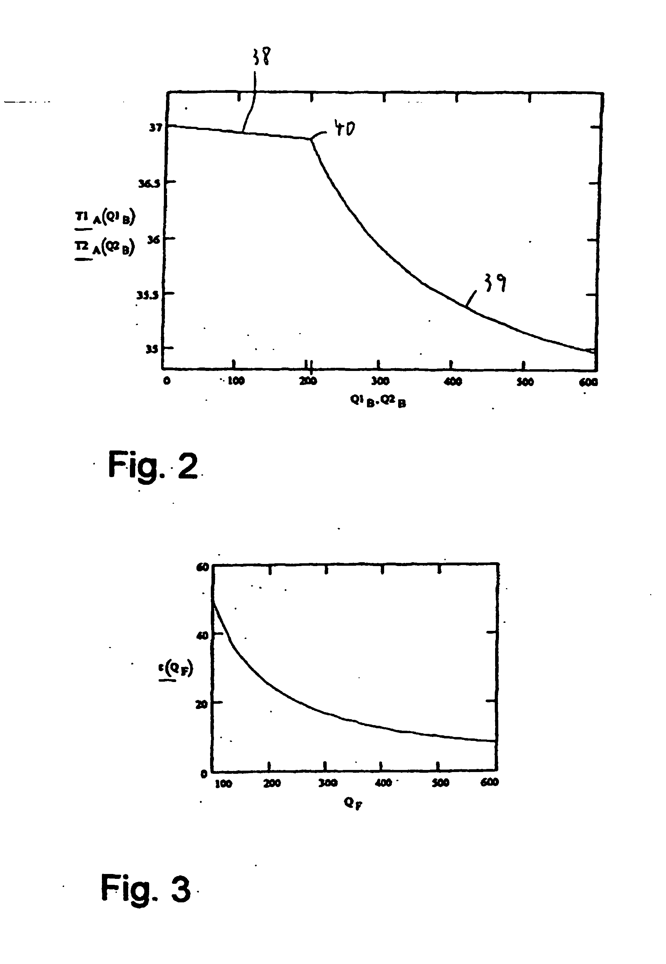

Fig. 2 zeigt die Bluttemperatur $T_A$ im arteriellen Zweig 20 des extrakorporalen Kreislaufs 9 als Funktion des extrakorporalen Blutflusses $Q_B$, wobei auf der horizontalen Achse der gängige Blutflußbereich bei der Hämodialyse von 100 bis 600 ml/min angegeben ist. An dem Punkt, an dem die Steigung der Kurve eine Unstetigkeitsstelle aufweist, ist der extrakorporale Blutfluß gleich dem Fistelfluß. Der Funktionsverlauf ist nach Gleichung (1) und (2) berechnet, wobei CO = 5 l/min, $Q_F$ = 200 ml/min, $T_B$ = 37°C und $T_V$ = 34°C gesetzt wurde.

Fig. 3 zeigt das Verhältnis der Steigungen der beiden Kurvenabschnitte der Funktion $T_A(Q_B)$ für den Schnittpunkt bei $Q_B = Q_F$. Abhängig vom Fistelfluß $Q_F$ ergeben sich Werte zwischen 50 ($Q_F$ = 100 ml/min) und 8,3 ($Q_F$ = 600 ml/min). Die Diskontinuität der Kurvensteigung kann also im gesamten Blutflußintervall von 100 bis 600 ml/min ausreichend scharf detektiert werden.

Zur Ermittlung des Fistelflusses wird die den extrakorporalen Blutfluß vorgebende Förderrate der Blutpumpe 26 ausgehend von einem vorgegebenen unteren Grenzwert von z.B. 100 ml/min bis zu einem oberen Grenzwert von z.B. 600 ml/min langsam erhöht, wobei der untere Grenzwert einem extrakorporalen Blutfluß $Q_B$ entspricht, der auf jeden Fall kleiner als der zu erwartende Fistelfluß $Q_F$ ist und der obere Grenzwert einem entsprechenden Blutfluß entspricht, der auf jeden Fall größer als der zu erwartende Fistelfluß ist. Die Temperatur $T_A$ des Bluts im arteriellen Zweig 20 des extrakorporalen Kreislaufs 9 wird während der Erhöhung des Blutflusses mit der arteriellen Meßeinrichtung 24 gemessen und die Werte des extrakorporalen Blutflusses und der Temperatur $T_A$ werden in der Speichereinheit 30 in zeitlicher Abfolge abgespeichert.

In der Recheneinheit 35 werden aus der abgespeicherten Folge von Wertepaaren die Differenzenquotienten berechnet. Die berechneten Differenzenquotienten der Wertepaare werden mit einem vorgegebenen Grenzwert verglichen. In der Recheneinheit 35 wird nun derjenige Wert des Blutflusses $Q_B$ im extrakorporalen Kreislauf bestimmt, nach dessen Überschreiten die Beträge der ermittelten Differenzenquotienten größer als ein vorgegebener Grenzwert sind, d.h. es wird derjenige Punkt bestimmt, an dem die in Fig. 2 dargestellte Kurve plötzlich stark abfällt. Während die Steigung des in Fig. 2 dargestellten linken Kurvenabschnitts nämlich klein und nahezu konstant ist, tritt in dem Fall, daß der Blutfluß $Q_B$ größer als der Fistelfluß $Q_F$ ist, eine plötzlich starke Erhöhung des Betrages der Steigung auf. Bei der Messung wird angenommen, daß der ermittelte Wert des Blutflusses $Q_B$ dem Fistelfluß $Q_F$ entspricht. Dieser Wert wird als Schätzwert für den Fistelfluß in der Speichereinheit 30 abgespeichert.

Um den Wert des Fistelflusses exakt, d.h. unbeeinflußt von eventuellen Fluktuationen des Blutflusses im Bereich des Fistelflusses, bestimmen zu können, werden in der Recheneinheit 35 die abgespeicherten Meßwertpaare für Blutflußwerte $Q_B$ kleiner als der Schätzwert für den Fistelfluß durch die erste Teilfunktion (1) angepaßt und die abgespeicherten Meßwertpaare für Blutflußwerte größer als der Schätzwert für den Fistelfluß werden durch die zweite Teilfunktion (2) angepaßt. Dabei ist es vorteilhaft, wenn Meßwertpaare aus einem kleinen Bereich (z.B. $\pm$ 30 ml/min) um den Schätzwert für den Fistelfluß ausgespart werden. Zur Berechnung der Parameter der beiden Gleichungen können die bekannten numerischen Verfahren herangezogen werden (z.B. Levenberg-Marquardt-Methode). Eine hinreichende Genauigkeit wird im allgemeinen auch dann erreicht, wenn anstelle von Gleichung (1) eine einfache lineare Funktion zur Darstellung des Kurvenverlaufs herangezogen wird. Anschließend berechnet die Recheneinheit 35 den Wert des extrakorporalen Blutflusses $Q_B$, der dem Fistelfluß $Q_F$ entspricht durch des Schnittpunktes 40 der beiden Teilfunktionen 38 und 39. Dieser Wert wird in der Speichereinheit 30 abgespeichert und mit der Anzeigeeinheit 37 angezeigt.

Zur Ermittlung der Körpertemperatur $T_B$ wird in der Recheneinheit der in Fig.2 durch Gleichung (1) dargestellte linke Kurvenabschnitt auf einen Blutflußwert von 0 extrapoliert ($T_B = T_A(0)$). Der Temperaturwert für einen Blutflußwert von 0, der sich durch eine Messung nicht ermitteln läßt, entspricht der Körpertemperatur $T_B$. Diese wird in der Speichereinheit 30 abgespeichert und auf der Anzeigeeinheit 37 zur Anzeige gebracht.

Nachdem der Fistelfluß $Q_F$ und die Körpertemperatur $T_B$ ermittelt und abgespeichert sind, wird in der Recheneinheit 35 das Herzminutenvolumen CO wie folgt berechnet:

$$CO = Q_F \frac{T_V - T_B}{T_A(Q_F) - T_B} \qquad (3)$$

Gleichung (3) folgt aus Gleichung (1) mit $Q_B = Q_F$.

Auch dieser hämodynamische Parameter wird auf der Anzeigeeinheit 37 angezeigt.

Die Steuereinheit 29 der erfindungsgemäßen Vorrichtung steuert die einzelnen Systemkomponenten nach einem vorgegebenen Programmablauf an, so daß der Blutfluß $Q_B$ nach Betätigung einer in Fig. 1 nicht dargestellten Taste zum Starten der Messung automatisch in dem Intervall von 100 bis 600 ml/min verändert wird, die Meßwerte mittels der Temperaturmeßeinrichtungen 24, 25 in den entsprechenden Intervallen aufgenommen und in der Speichereinheit 30 abgespeichert werden und aus den Meßwerten die hämodynamischen Parameter in der Recheneinheit 35 berechnet und auf der Anzeigeeinheit 37 angezeigt werden.

Das Verfahren erfordert keine wesentlichen Änderungen am extrakorporalen Kreislauf und am Sicherheitskonzept der Dialysevorrichtung. Die Injektion einer Indikatorlösung ist nicht erforderlich. Zwar sind Fistelflüsse nur im Bereich des einstellbaren Blutflusses meßbar (ca. 100 bis 600 ml/min), dies bedeutet in der Praxis allerdings keine Einschränkung. Falls der Fistelfluß größer als der einstellbare Blutfluß ist, ergeben sich nämlich für die Therapie keine Probleme.

**Patentansprüche**

1. Vorrichtung zur Ermittlung hämodynamischer Parameter während einer extrakorporalen Blutbehandlung, bei der Blut über den arteriellen Zweig (20) des extrakorporalen Kreislaufs (9), der mit dem arteriellen Teil (21) einer Fistel (6) in Fluidverbindung steht, in einen Dialysator (10) oder Filter der Blutbehandlungsvorrichtung (7) gelangt und über einen venösen Zweig (22) des extrakorporalen Kreislaufs (9), der mit dem venösen Teil (23) der Fistel (6) in Fluidverbindung steht, zurückgeführt wird, wobei in den extrakorporalen Kreislauf (9) eine Blutpumpe (26) geschaltet ist, mit

- einer arteriellen Meßeinrichtung (24) zum Messen einer physikalischen oder chemischen Kenngröße $X_A$ des Blutes im arteriellen Zweig (20) des extrakorporalen Kreislaufs (9), wobei die physikalische oder chemische Kenngröße eine Eigenschaft des Blutes ist, die in dem im venösen Zweig (22) des extrakorporalen Kreislaufs (9) fließenden Blut einen anderen Wert als in dem intrakorporal in die Fistel (6) fließenden Blut hat,
- einer Steuereinheit (29) zum Verändern der Förderrate der Blutpumpe (26),
- einer Speichereinheit (30), die derart ausgebildet Ist, daß die Werte des durch die Förderrate der Blutpumpe (26) vorgegebenen Blutflusses $Q_B$ und die Werte der mit der arteriellen Meßeinrichtung (24) gemessenen physikalischen oder chemischen Kenngröße $X_A$ im arteriellen Zweig (20) des extrakorporalen Kreislaufs (9) abspeicherbar sind, und
- einer Recheneinheit (35), die derart ausgebildet ist, daß aus der abgespeicherten Folge von Wertepaaren der physikalischen oderchemischen Kenngröße $X_A$ des Blutes im arteriellen Zweig (20) des extrakorporalen Kreislaufs (9) und des extrakorporalen Blutflusses $Q_B$ derjenige Wert des Blutflusses bestimmbar ist, nach dessen Überschreiten der Betrag der Änderung der physikalischen oder chemischen Kenngröße in einem bestimmten Blutflußintervall größer als ein vorgegebener Grenzwert ist, wobei aus dem ermittelten Blutflußwert der Fistetfluß $Q_F$ ermittelbar ist.

2. Vorrichtung nach Anspruch 1, **dadurch gekennzeichnet, daß** die Recheneinheit (35) derart ausgebildet ist, daß aus dem ermittelten Fistelfluß $Q_F$ der mittlere Wert $X_B$ derphysikalischen oder chemischen Kenngröße im venösen Blut und/oder das Herzminutenvolumen CO ermittelbar sind.

3. Vorrichtung nach Anspruch 1 oder 2, **dadurch gekennzeichnet, daß** die im arteriellen Zweig (20) des extrakorporalen Kreislaufs (9) zu messende physikalische oder chemische Kenngröße des Blutes die Bluttemperatur $T_A$ und die arterielle Meßeinrichtung (24) eine Temperaturmeßeinrichtung ist.

4. Dialysevorrichtung mit einer Vorrichtung nach einem der Ansprüche 1 bis 3.

**Claims**

1. Device for determining haemodynamic parameters during extracorporeal blood treatment, in which blood is fed into a dialyser (10) or filter of the blood treatment device (7) via the arterial branch (20) of the extracorporeal circuit (9) which is in liquid communication with the arterial part (21) of a fistula (6), and is returned via a venous branch (22) of the extracorporeal circuit (9) which is in liquid communication with the venous part (23) of the fistula (6), a blood pump (26) being incorporated in the extracorporeal circuit (9), with

- an arterial measuring device (24) for measuring a physical or chemical characteristic magnitude $X_A$ of the blood in the arterial branch (20) of the extracorporeal circuit (9), the physical or chemical characteristic magnitude being a property of the blood which in the blood flowing in the venous branch (22) of the extracorporeal circuit (9) has a value other than in the blood flowing intracorporeally into the fistula (6),
- a control unit (29) for varying the delivery rate of the blood pump (26),
- a storage unit (30) which is designed so that the values of the blood flow $Q_B$ predetermined by the delivery rate of the blood pump (26) and the values of the physical or chemical characteristic magnitude $X_A$ in the arterial branch (20) of the extracorporeal circuit (9), measured with the arterial measuring device (24) can be stored and
- a computing unit (35) which is designed in such a way that it is possible, from the stored sequence of value pairs of the physical or chemical characteristic magnitude $X_A$ of the blood in the arterial branch (20) of the extracorporeal circuit (9) and the extracorporeal blood flow $Q_B$, to determine the value of the blood flow at which, after the latter has been exceeded, the amount of the change in the physical or chemical characteristic magnitude in a specific blood interval is greater than a predetermined threshold value, whereby the fistula flow $Q_F$ can be determined from the ascertained blood flow value.

2. Device according to claim 1, **characterised in that** the computing unit (35) is designed in such a way that the mean value $X_B$ of the physical or chemical characteristic magnitude in the venous blood and/or the heart minute volume CO can be determined from the determined fistula flow $Q_F$.

3. Device according to claim 1 or 2, **characterised in that** the physical or chemical characteristic magnitude of the blood to be measured in the arterial branch (20) of the extracorporeal circuit (9) is the blood temperature $T_A$ and the arterial measuring device (24) is a temperature measuring device.

EP 0 773 035 B2

**4.** Dialysis device with a device according to one of claims 1 to 3

**Revendications**

**1.** Dispositif de détermination de variables hémodynamiques pendant un traitement extracorporel du sang, dans lequel le sang est amené à un dialyseur (10) ou un filtre du dispositif de traitement du sang (7) via la ligne artérielle (20) du circuit extracorporel (9), qui est en couplage de fluide avec la partie artérielle (21) d'une fistule (6) et ramené via une ligne veineuse (22) du circuit extracorporel (9) qui est en couplage de fluide avec la partie veineuse (23) de la fistule (6), une pompe à sang (26) étant raccordée au circuit extracorporel (9), avec

- un dispositif (24) de mesure d'une variable physique ou chimique $X_A$ du sang dans la ligne artérielle (20) du circuit extracorporel (9), la variable physique ou chimique étant une propriété du sang qui a, dans le sang s'écoulant dans la ligne veineuse (22) du circuit extracorporel (9), une valeur différente de celle du sang s'écoulant de façon intracorporelle dans la fistule (6),
- une unité de commande (29) permettant de modifier le débit de la pompe à sang (26),
- une unité d'enregistrement (30) agencée pour que les valeurs du flux sanguin $Q_B$ prédéfinies par le débit de la pompe à sang (26) et les valeurs de la variable physique ou chimique $X_A$ dans la ligne artérielle (20) du circuit extracorporel (9), mesurée avec le dispositif de mesure de la pression artérielle (24), puissent être mises en mémoire, et
- une unité de calcul (35) agencée pour que cette valeur du flux sanguin soit définissable d'après la suite mémorisée des paires de valeurs de la variable physique ou chimique $X_A$ du sang dans la ligne artérielle (20) du circuit extracorporel (9) et du flux sanguin extracorporel $Q_B$, après le dépassement de laquelle la valeur de la modification de la variable physique ou chimique est supérieure à une valeur-limite prédéfinie dans un intervalle du flux sanguin défini, le flux dans la fistule $Q_F$ étant calculable à partir de la valeur du flux sanguin déterminée.

**2.** Dispositif selon la revendication 1, **caractérisé en ce que** l'unité de calcul (35) est agencée pour que la valeur moyenne $X_B$ de la variable physique ou chimique dans le sang veineux et/ou le débit cardiaque CO soit calculable à partir du flux de la fistule $Q_F$ déterminé.

**3.** Dispositif selon la revendication 1 ou 2, **caractérisé en ce que** la variable physique ou chimique du sang à mesurer dans la ligne artérielle (20) du circuit extracorporel (9) est la température sanguine $T_A$ et **en ce que** le dispositif de mesure de la pression artérielle (24) est un dispositif de mesure de la température.

**4.** Dispositif de dialyse avec un dispositif selon l'une des revendications 1 à 3.

9

Fig. 1

Fig. 2

Fig. 3